# EUROPEAN PATENT APPLICATION

(11) **EP 0 873 755 A1**
(43) Date of publication of application: **28.10.1998**
(21) Application number: 98400983.7
(22) Date of filing: 22.04.1998
(51) Int. Cl.: A61K 39/40, A61K 39/02, A23L 1/00, C07K 16/02

(54) **Prevention and treatment of enterohemorrhagic E. coli infection**

(30) Priority: 23.04.1997 JP 106489/97
(71) Applicant: Ghen Corporation, Gifu-shi Gifu-ken (JP)
(72) Inventor: Kodama, Yoshikatsu, Gifu-shi, Gifu-ken (JP); Yokoyama, Hideaki, Kakamigahara-shi, Gifu-ken (JP); Koga, Yasuhiro, Isehara-shi, Kanagawa-ken (JP); Aiba, Yuji, Hadano-shi, Kanagawa-ken (JP); Tanaka, Kazuo, Atsugi-shi, Kanagawa-ken (JP)
(74) Representative: Schrimpf, Robert

(57) **Abstract**

The present invention provides a pharmaceutical composition for use in preventing or treating an infectious disease caused by enterohemorrhagic E. coli, and a food for preventing the disease. The composition comprises as an active ingredient specific antibodies against enterohemorrhagic E. coli, the antibodies being obtained from eggs laid by hens which have been immunized against whole cell enterohemorrhagic E. coli or toxoid of vero toxin produced by the bacterium as an antigen. At least one bacterium selected from lactic acid bacterium and bifid bacterium may be administered along with the above antibodies.

## Description

The present invention relates to the prevention and treatment of infectious diseases caused by infection with enterohemorrhagic Escherichia coli (hereinafter referred to as EHEC), and particularly to the prevention and treatment of hemorrhagic colitis caused by EHEC 0157 and complications thereof such as hemolytic uremic syndrome and central nervous disorders.

EHEC 0157 was first isolated in a mass outbreak of infectious diseases in 1982 in the United States Since then, the incidence of infectious disease caused by this bacterium has increased mainly in North America and Europe. Especially in England and United States, this infectious disease is regarded as an "Emerging Infectious Disease" which requires emergency monitoring, and preventive measures against are considered very important.

EHEC infection causes hemorrhagic colitis accompanied by diarrhea and hematochezia and sometimes causes complications such as hemolytic uremic syndrome (HUS) and disorders in the central nervous system. Vero toxin produced by EHEC is closely associated with these disorders, and therefore it is an important key for treatment of EHEC infection to develop a method for neutralizing vero toxin.

Methods for the treatment of EHEC infectious diseases proposed so far are explained below.

### (1) Treatment using antibiotics (Japanese Journal of Bacteriology, 52(1), 2016,2017 and 2018, 1997)

The use of antibiotics has the problem of increasing antibiotic-resistant strains. It is said that 95.9% of the patients with EHEC infection which was rampant in 1996 mainly in Sakai City, Japan were treated with antibiotics. It has been reported that fosfomycin (FOM) was the most widely used antibiotic at that time (FOM comprised 84% of the antibiotics used.) According to subsequent studies, FOM cannot suppress the production of vero toxin very well. On the contrary, FOM causes release of vero toxin bound to bacterial cells as a result of killing EHEC cells. Thus, there are problems with administering the antibiotic FOM to patients.

### (2) Use of commercially available human immunoglobulin preparations

Immunoglobulin preparations have been administered for treatment of patients with EHEC infection, but their effects are not clear. Komiya et al. (Japanese Journal of Bacteriology, 52(1), 3124, 1997) sampled at random 13 lots of γ-globulin preparations for intravenous injection from 5 manufacturers, and measured neutralizing-antibody titer level against cytotoxic effect on vero cells and mouse lethal toxicity to see whether the preparations have antibodies which neutralize EHEC VT1 or VT2.

Also, Takeda examined samples of γ-globulin preparations made from imported blood for their ability to neutralize EHEC VT1 or VT2. He reported that the preparations neutralized VT1, but did not neutralize VT2, which affects adversely symptoms ( Newsflash of Jiji Press, February 21, 1997).

From the above, it is likely that the use of human immunoglobulin preparations is of little effect on the treatment of EHEC infections.

### (3) Use of bovine colostral immunoglobulins

Pirro,F. et al. (Veterinary Microbiology 43, 131-141, 1995) collected 225 random samples of bovine colostrum and 40 random samples of sera in Germany, and examined whether each of these colostrums and sera has antibodies against EHEC VT1 or VT2. It was found that 69.3% of the colostrums had anti-toxin antibodies against VT1 and 14.7% had antibodies against VT1 and VT2. The same tendency could be seen in the sera. The isotype of the toxin-neutralizing antibody was IgG1. It was concluded from these data that bovines which are conventionally bred in Germany have EHEC, and therefore people who have meat and milk from the polluted bovines would have a higher risk than expected. Similar epidemiological data have been reported in Canada.

Lissner,R. et al. (Infection 24, No.5, 44-49, 1996) collected and pooled bovine colostrum from at least 100 bovines bred conventionally in German within 10 hours after parturition. They obtained whey through conventional delipidation and removal of casein and spray dried the whey to prepare 2-5 kg of whey powder (Lactobin ™) from about 100 kg of colostrum. Using the method of Pirro et al. (1995), the obtained whey powder was examined in vitro as to whether antibodies (IgG1) contained therein specifically neutralized EHEC VT1 and VT2. It was found that the whey powder contained a high titer of antibodies which neutralized VT1 and VT2. Lissner,R. et al. stated that this result suggests the possibility that diarrhea and HUS conditions are lessened by orally administering pooled whey to patients with EHEC infection. However, it was not confirmed experimentally that the dried whey can be used for therapeutical purposes. That is to say, it is not clear whether death by EHEC infection can be prevented, colonization of EHEC in the intestines can be prevented, or VT1 and VT2 in the intestines can be neutralized when this dried whey is orally administered to EHEC infection model animals.

Additionally, the above method is inefficient for preparing antibodies since the antibody titer in colostrum generally decreases rapidly with time after parturition, and therefore the period for collecting is limited, and desired antibodies can be recovered only in small amounts. Furthermore, only bovines having EHEC can produce colostrum containing antibodies against Vt1 and VT2, but it increases the danger of infection to use colostrum which may be polluted with EHEC as materials for recovering antibodies, as pointed out by Pirro,F. (1995). Accordingly, the whey powder obtained by the above-mentioned method is not suitable for therapeutic purposes in humans.

As mentioned above, there have not been proposed effective methods for treatment of enterohemorrhagic E.coli 0157 infection, which is currently a major problem.

Antibiotic therapy results in a release of vero toxin and an increase of antibiotic-resistant strains. Human immunoglobulin preparations have no neutralizing activity against vero toxin VT2. Colostrum from bovines polluted with EHEC has anti-EHEC antibodies, but the danger of infection with EHEC will increase.

The object of the present invention is to provide effective and safe pharmaceutical compositions for treatment and prevention of enterohemorrhagic E.coli 0157 infection.

The present inventors previously developed a method for preparing specific antibodies against a particular antigen in large amounts and inexpensively using chicken eggs, and applied the method to the preparation of specific antibodies against enterotoxigenic E.coli (ETEC) which causes colibacillosis in suckling pigs and calves. The obtained antibodies were found to be effective for treatment and prevention of colibacillosis (Japanese Patent Application Kokai No. 7-53669). Specifically, hens were immunized with pilus antigens (987p, K88 and K99 antigen), which are adherence factors of ETEC, and antibodies which had transferred to egg yolk were recovered. The mechanism of diarrhea caused by ETEC infection is that the bacteria colonize the intestine and grow there, and enterotoxins (LT, ST) produced by the bacteria cause loss of electrolytic balance. The object of the above-mentioned method is to prevent and treat colibacillosis by utilizing specific antibodies against adherence factors which are essential for colonization of this bacterium in the intestine.

On the other hand, the factor causing hemorrhagic colitis, HUS or central nervous disorders is vero toxin in EHEC infection. Adherence factors have not been found in EHEC, unlike in ETEC. Accordingly, prevention and emergency treatment of EHEC infection may be possible by developing oral passive immunization therapy which enables vero toxin produced in the intestine by EHEC infection to be neutralized before binding to targeted cells.

However, an EHEC infection model is essential in order to evaluate the effects of obtained antibodies. Since conventional mice have normal bacterial flora in the intestines, mice cannot be infected with EHEC merely by oral inoculation with an EHEC culture. When mice are pretreated with streptomycin sulfate and mitomycin C, they can be infected with EHEC by oral inoculation and exhibit clinical manifestations such as lethargy, weight loss, paralysis of four legs, and death by infection. However, pretreatment with these substances is not suitable for testing the efficacy of drugs since it may affect the biological protection system.

The present inventors have found that oral inoculation of EHEC culture to germ-free mice enables infection with EHEC without pretreatment and causes lethal conditions, and developed an EHEC infection model suitable for evaluating the efficacy of drugs for prevention or treatment of EHEC infection. The present inventors completed the present invention based on the discovery using such an infection model that eggs laid by hens immunized with inactivated EHEC contain sufficient antibodies which are able to neutralize vero toxin and that these eggs are effective in prevention and treatment of EHEC infection.

The present invention provides a pharmaceutical composition for use in prevention or treatment of infectious diseases caused by enterohemorrhagic E.coli, which contains as an active ingredient specific antibodies obtained from eggs laid by hens which have been immunized against enterohemorrhagic E.coli cells or toxoid of vero toxin produced by EHEC as an antigen. The present invention also provides a food containing as an additive the above-described antibodies. In a preferred embodiment, the present invention provides a pharmaceutical composition for use in prevention or treatment of enterohemorrhagic E.coli 0157, which contains as an active ingredient specific antibodies obtained from eggs laid by hens which have been immunized against inactivated cells of enterohemorrhagic E.coli 0157 as an antigen. The specific antibodies are capable of agglutinating specifically E.coli 0157 cells and have neutralizing activity against vero toxin produced by the bacterium.

The above-mentioned antibodies obtained from eggs laid by hens immunized with EHEC cells can be used along with at least one bacterium selected from lactic acid bacteria and bifid bacteria in prevention or treatment of infectious diseases caused by EHEC, especially EHEC 0157.

In order to produce the specific antibodies against EHEC, hens are immunized against an antigen. Antigens used to immunize hens include inactivated cells obtained by culturing EHEC such as EHEC 0157 T001, T002, T003, T004 and T005 and adding formalin to the culture. Alternatively, toxoid antigens obtained by adding Polymyxin B to a cell suspension (1x10¹⁰-1x10¹¹CFU/ml)(PBS, pH7.2) to lyze the cells, extracting vero toxin from the cells and adding formalin to vero toxin to prepare a toxoid can be used.

The immunization of hens against an antigen may be performed by inoculation with the antigen by an appropriate route such as subcutaneous or intramuscular injection. Preferably, a suitable adjuvant is administered in conjunction with the antigen to enhance the immunization. Adjuvants useful for this purpose are Freund's complete (incomplete) adjuvant (Difco), Cholera toxin BB (Sigma), Titer Max (CytRx Corp.), etc.

The dose of the antigen is determined depending on the type of the antigen and adjuvant and the administration route in such a manner that an immune state is induced in the hen without producing excessive toxicity of the antigen in the hen.

Usually, within a few weeks after the inoculation (initial immunization), the hen becomes sensitive to the antigen, i.e., immunized against the antigen. Specific antibodies against the antigen are produced within the body of the hen, and eggs laid by the hen, especially the yolks of the eggs, contain the specific antibodies.

After the initial immunization of the hen against the antigen, one or more boosters at an appropriate dose level may be administered in order to maintain a high antibody titer in the hen.

The presence and titer level of the specific antibodies against the antigen in the hen and in eggs of the hen can be confirmed by any known method known to those skilled in the art of immunological assays, such as ELISA or a method using an aggulination reaction.

A titer of anti-vero toxic antibody can be determined by the method of Pirro et al. (1995) utilizing a suspension of vero cells. After it is confirmed that an adequate titer of the specific antibodies is present in eggs laid by the immunized hen, eggs laid by the hen are collected and the desired antibodies are recovered.

The specific antibodies of the present invention may be prepared from the overall ovum or the yolk of the eggs. Most antibodies are contained in the yolk of an egg, and usually the yolk is separated from the egg for use in the production of the antibodies. In some cases, overall ovum of the egg may be used.

The overall ovum or the yolk of the egg may be used without fractionation. Alternatively, the overall ovum or the yolk of the egg may be subjected to fractionation or purification. For example, a delipidation procedure may be carried out by suitable methods such as methods using hydroxypropylmethylcellurose phthalate, polyethyleneglycol, etc. to remove lipid components from the yolk. If desired, further purification may be carried out by any known method, including known purification procedures of proteins such as salting out with ammonium sulfate or sodium sulfate, or cold ethanol precipitation, etc.

The overall ovum or the yolk of the egg, without fractionation or with fractionation or purification, may be used directly or it may be processed. In a preferred embodiment, the overall ovum or the yolk may be stirred or homogenized into an emulsion and dried to form a powder by conventional techniques such as spray drying or lyophilizing. Thus, various forms of antibodies may be used depending on the purpose.

The obtained antibodies which are specific for EHEC have sufficient neutralizing activity against vero toxin even if EHEC whole cells are used as an antigen. Furthermore, it was demonstrated by experiments using EHEC infection model animals that the oral administration of the specific antibodies of the present invention decreased the number of EHEC cells in the intestines and suppressed the production of vero toxin, thereby decreasing mortality.

A greater protective effect against EHEC infection is obtained when the specific antibodies from eggs against EHEC are orally administered along with at least one bacterium selected from lactic acid bacteria and bifid bacteria. With respect to the administration of lactic acid bacteria, Ogawa et al. (Japanese Journal of Bacteriology 52(1), pp157, 2006 (1997)) and Koga et al. Japanese Journal of Bacteriology 52(1), pp301, 3126 (1997)) reported that lactic acid bacteria suppress the growth of EHEC and have an activity of neutralizing vero toxin to some extent, and it was supposed that this was due to an organic acid such as acetic acid or lactic acid produced by lactic acid bacteria. However, a remarkable efficacy could not be expected when lactic acid bacteria is used along with the specific antibodies of the present invention.

Thus, the specific antibodies of the present invention can be used in prevention and treatment of EHEC infection, and can be used as an additive to foods for prevention of EHEC infection, alone or in combination with lactic acid bacteria and/or bifid bacteria.

Examples of lactic acid bacteria used in the present invention include Lactobacillus acidophilus, Lactobacillus lactis, Lactobacillus bulgaricus, Lactobacillus casei, Enterococcus faecalis, Enterococcus faecium, etc. Examples of bifid bacteria used in the present invention include Bifidobacterium breve, Bifidobacterium bifidum, Bifidobacterium thermophilum, Bifidobacterium longum, Bifidobacterium infantis, Bifidobacterium adolescentis, etc.

When the specific antibodies of the present invention are used in preventing or treating infectious diseases, the antibodies can be conveniently administered in the form of a pharmaceutical composition containing the antibodies and a pharmaceutically acceptable carrier or diluent. In preparing a pharmaceutical composition, antacids (such as sodium hydrogencarbonate, magnesium carbonate, precipitated calcium carbonate, or magnesium metasilicate aluminate) may be added to the pharmaceutical composition. The preparation of the pharmaceutical composition may be performed by any known methods.

The administration of the pharmaceutical composition for use in prevention or treatment of the infectious disease may be by an oral route. The dosage of the antibodies of the present invention is selected according to the usage, purpose and conditions of symptoms. When the antibodies are administered for preventing EHEC infection, preferably 0.25-20.0 mg/kg of the purified antibodies may be administered per day. In case of treating infectious diseases caused by EHEC, 1.25-125 mg/kg of the purified antibodies may be preferably administered. When the bacterium selected from lactic acid bacteria and bifid bacteria is administered in combination with the antibodies, they may be administered preferably in an amount of 10⁷-10¹⁰ viable cells per day.

When the antibodies of the present invention are used as an additive to a food for prevention of EHEC infection, the antibodies are added to a food in an amount of 0.01-0.1 wt %, and preferably 0.05 wt % of the food as purified antibodies. The bacterium selected from lactic acid bacteria and bifid bacteria may be added to a food in an amount of 10⁶-10⁹ viable cells per 1 gram of the food.

### Example 1

This example illustrates the preparation of egg powder containing specific antibodies from eggs of immunized hens.

### Preparation of inactivated antigen

EHEC T001 was isolated from diarrhea feces of the patient with EHEC, and was inoculated in 20 l of Tripticase soy broth. After culturing aerobically for 18 hours, formalin was added to this culture in an amount of 3% of the culture for inactivation of the cells. After confirming that cells were inactivated in the culture, the culture was centrifuged at 12,000xg for 20 minutes and washed three times with PBS (pH7.2) to obtain an antigen for immunization.

### Immunization

The inactivated cells were adjusted with PBS to contain 10¹⁰-10¹¹ CFU/ml and mixed with an oil adjuvant to prepare an immunogen. The immunogen was injected into the pectoral muscles of White Leghorn hens 16-18 weeks old at a dose of 0.5 ml per injection (initial injection). Eight weeks after the initial immunization, the same immunogen was injected as a booster administration in the same manner and in the same dose.

The antibody titer of egg yolk was determined using microplate 96 wells as follows. The yolk was separated from the albumen of each egg, and an equal weight of PBS was added to the yolk. To this mixture, an equal volume of chloroform was added and then the mixture was shaken. After centrifuging this mixture, supernatant (water soluble fraction) was recovered as a sample for determining the antibody titer. To each 0.25 ml of serial 2-fold dilutions of the sample in PBS, an equal volume of antigen for agglutination reaction which had been adjusted to have an O.D. value of 0.45 at 560 nm was added and the mixture was shaken. After the surface of the microplate was sealed, sensitization was conducted at 37°C for 2 hours. The microplate was allowed to stand at 4°C for about 18 hours and the presence of agglutination was evaluated. The agglutination antibody titer was expressed as the reciprocal of the highest dilution of the sample that showed complete agglutination. The sample from each hen had an antibody titer of 1,280-5,120 two weeks after booster injection, and the eggs began to be collected.

### Preparation of antibodies from egg yolk

After the immunized eggs were washed and disinfected, the yolk was separated from the albumen of each egg, and combined yolk from a plurality of eggs was divided into groups of 8 kg and stored below -20°C until used. The purification was carried out as follows: To 7.5 kg of the yolk as a starting material was added a 10-fold amount (by weight) of distilled water to cause delipidization. To the supernatant was added ammonium sulfate to produce 40% saturation. The mixture was stirred and centrifuged to obtain pellets. The pellets were dissolved in saline, and ethanol at -20°C was added gradually to produce a final concentration of 50% while dissolving and shaking was carried out. After centrifugation at 4°C, the pellets were dissolved in saline and filtered to remove bacteria and lyophilized. As a result, about 15 g of a pale yellowish white powder was obtained from 7.5 kg of egg yolk. The recovery rate of antibodies was 50%, the purity of IgG was 95%, and the content of water was 2%.

### Experiment 1

This experiment was conducted to study the ability of egg yolk antibody powder to neutralize vero toxin (VT1, VT2). Vero toxin-neutralizing ability was determined by the method of Pirro et al (1995), with some modification as follows. 1 g of antibody powder was dissolved in 10 ml of sterilized distilled water and then serial 2-fold dilutions of the solution were made using PBS. To each well was added an equal volume of vero toxin adjusted to have a toxin unit of 10 CD ₅₀, and sensitization was carried out at 37°C for 60 minutes. Then, to each well was added a suspension of vero cells adjusted to contain 5x10⁴ cells, and incubation was carried out at 37°C for 96 hours in a CO₂ incubator. The anti-toxin antibody titer was expressed as the reciprocal of the highest dilution that showed inhibition of cytotoxic effect.

The egg yolk antibody powder had a neutralizing antibody titer of 2,560 against VT1 and of 640 against VT2. The agglutination antibody titer was 20,480. Therefore, the prepared egg yolk antibody powder can neutralize both types of vero toxins and agglutinate cells.

### Experiment 2

The efficacies were studied in this experiment when egg yolk antibodies, mixed bacterial culture, and a combination of thereof were used in an EHEC infection model of germ-free mice which had been orally inoculated with EHEC culture with which they were to be infected. The mice were divided into 4 groups, with each group consisting of 10 germ-free BALB/c male mice 4 weeks old. In Group I, each mouse was orally administered 1 g of antibody powder from egg yolk in 10 ml of sterilized distilled water. Each mouse in Group II was orally administered a mixed bacterial culture of human Lactobacillus acidophilus (JCM 1028), Bifidobacterium breve (JCM 1192T), and Bifidobacterium bifidum (JCM 1209) obtained by culturing at 37°C for 18 hours in Briggs liver broth. Each mouse in Group III was orally administered a 1:1 mixture of the above-mentioned antibody solution and the mixed culture. In Group IV, each mouse was orally administered sterilized Briggs liver broth. The dose was 0.5 ml per mouse per day and the administration was once a day for three consecutive days.

Three hours after the first administration of antibodies or other materials, each mouse was challenged with EHEC T001 (O157;H7) at a dose of 1x10⁷ CFU. Clinical responses were observed for seven days after challenging. Mice which were alive and dying were sacrificed. Each mouse was examined for the number of EHEC cells in the colon and the presence of vero toxin using a kit for detection of vero toxin.

Table 1 shows the number of cells in the colon, the presence of vero toxin, paralysis of four legs (%), and mortality rate in the administrated groups and the control group. When comparing the administered groups and the control group, Group III (administered the combination of the antibodies from eggs and mixed culture of bacteria) had the highest efficacy and Group I (antibodies from eggs) had a rather high efficacy. A low efficacy was found in Group II.

It is noteworthy that in Group III, little vero toxin was detected although EHEC in the colon was not completely eliminated. It is believed that this is because egg yolk antibodies of the present invention were effective for protecting against clinical manifestations (paralysis of four legs), and decreasing mortality rate.

It is supposed that vero toxin produced by growth of EHEC was neutralized with administrated antibodies in mice since vero toxin was neutralized in vitro with antibody powder from egg yolk used in this experiment. On the other hand, it has been reported that lactic acid bacteria have an activity of inhibiting the growth of EHEC and neutralizing vero toxin to some extent in vitro and in vivo. However, the mixed bacterial culture had an insufficient effect by itself, as is apparent from Table 1. However, the combination of the mixed culture and the specific antibodies of the present invention could suppress disorders caused by infection with EHEC 0157 and remarkably decrease the mortality rate in mice.

**Table 1**

| Effects of egg yolk antibodies and mixed bacterial culture in mice infected with EHEC | | | | | |
|---|---|---|---|---|---|
| Group | Number of mice used | In the colon | | Paralysis(%) | Number of dead/total (%) |
| | | Number of cells (Lg10)/g | Vero toxin | | |
| I | 10 | 8.2 ± 0.7 | ±∼+ | 0/10(0) | 2/10(20) |
| II | 10 | 9.3 ± 0.3 | +∼++ | 6/10(60) | 5/10(50) |
| III | 10 | 7.6 ± 0.8 | ±∼- | 0/10(0) | 1/10(10) |
| IV | 10 | 10.1 ± 0.2 | +++ | 10/10(100) | 9/10(90) |

As is apparent from the above, the specific antibodies obtained from eggs laid by hens which have been immunized against whole cells or toxoid of vero toxin of EHEC as an antigen are effective in the prevention or treatment of EHEC infection, especially EHEC 0157 infection. The specific antibodies are prepared using eggs of hens, and therefore specific antibodies can be produced in large amounts and inexpensively by simple procedures and the obtained egg yolk antibodies are safe and without side effects.

## Claims

1. A pharmaceutical composition useful in the prevention or treatment of infectious diseases caused by enterohemorrhagic Escherichia coli, comprising as an active ingredient specific antibodies against enterohemorrhagic E. coli, and a pharmaceutically acceptable carrier or diluent, the antibodies being obtained from eggs laid by hens which have been immunized against whole cell enterohemorrhagic E. coli or toxoid of vero toxin produced by the bacterium as an antigen.

2. A pharmaceutical composition useful in the prevention or treatment of infectious diseases caused by enterohemorrhagic Escherichia coli 0157, comprising as an active ingredient specific antibodies against enterohemorrhagic E. coli 0157 and a pharmaceutically acceptable carrier or diluent, the specific antibodies being obtained from eggs laid by hens which have been immunized against inactivated cells of enterohemorrhagic E. coli 0157 as an antigen, and being capable of agglutinating the bacterial cells specifically and having neutralizing activity against vero toxin produced by the bacterium.

3. A pharmaceutical composition according to Claim 1 or 2, which is orally administrable.

4. A pharmaceutical composition useful in the prevention or treatment of infectious diseases caused by enterohemorrhagic Escherichia coli, comprising (a) specific antibodies against enterohemorrhagic E. coli, (b) at least one bacterium selected from lactic acid bacteria and bifid bacteria, and (c) a pharmaceutically acceptable carrier or diluent, the antibodies being obtained from eggs laid by hens which have been immunized against whole cell enterohemorrhagic E. coli or toxoid of vero toxin produced by the bacterium as an antigen.

5. A pharmaceutical composition according to Claim 4, wherein the enterohemorrhagic E. coli is enterohemorrhagic E. coli 0157.

6. A food useful for the prevention of infectious diseases caused by enterohemorrhagic E. coli, containing specific antibodies against enterohemorrhagic E. coli, the antibodies being obtained from eggs laid by hens which have been immunized against whole cell enterohemorrhagic E. coli or toxoid of vero toxin produced by the bacterium as an antigen.

7. A food according to Claim 6 wherein the antibodies are contained in an amount of 0.01 - 0.1 wt% of the food as purified antibodies.

8. A food useful for the prevention of infectious diseases caused by enterohemorrhagic E. coli, containing specific antibodies against enterohemorrhagic E. coli, and at least one bacterium selected from lactic acid bacteria and bifid bacteria, the antibodies being obtained from eggs laid by hens which have been immunized against whole cell enterohemorrhagic E. coli or toxoid of vero toxin produced by the bacterium as an antigen.

9. A food according to Claim 8, wherein the antibodies are contained in an amount of 0.01 - 0.1 wt% of the food as purified antibodies, and the bacterium are contained in an amount of 10⁶-10⁹ viable cells per 1 gram of the food.

10. A food according to Claim 6 or 8, wherein the enterohemorrhagic E. coli is enterohemorrhagic E. coli 0157.

11. A method for preventing or treating an infectious disease caused by enterohemorrhagic Escherichia coli, comprising orally administering specific antibodies against enterohemorrhagic E. coli in an effective amount for preventing or treating the infectious disease, the specific antibodies being obtained from eggs laid by hens which have been immunized against whole cell enterohemorrhagic E. coli or toxoid of vero toxin produced by the bacterium as an antigen.
